# EUROPEAN PATENT SPECIFICATION

(11) **EP 0 635 804 B1**
(45) Date of publication and mention of the grant of the patent: **27.02.2002**
(21) Application number: 94202061.1
(22) Date of filing: 15.07.1994
(51) Int. Cl.: G06T 7/00, H04N 5/32

(54) **Image processing method and device for performing that method**
Bildverarbeitungsverfahren und Vorrichtung zur Durchführung dieses Verfahrens
Méthode de traitement d'image et dispositif d'exécution de cette méthode

(30) Priority: 22.07.1993 EP 93401908; 22.07.1993 FR 9309052
(43) Date of publication of application: 25.01.1995
(73) Proprietor: Koninklijke Philips Electronics N.V., 5621 BA Eindhoven (NL); LABORATOIRES D'ELECTRONIQUE PHILIPS, 94450 Limeil-Brévannes (FR)
(72) Inventor: van Eeuwijk, Alexander Henricus Waltherus, NL-5656 AA Eindhoven (NL); Lobregt, Steven, NL-5656 AA Eindhoven (NL); Florent, Raoul, NL-5656 AA Eindhoven (NL); Breitenstein, Jacques, NL-5656 AA Eindhoven (NL)
(74) Representative: Lottin, Claudine

(56) References cited:
- EP-A- 0 309 813
- WO-A-92/11609
- US-A- 3 706 851
- US-A- 4 961 425

## Description

The invention relates to an image processing method formed by x-ray imaging, and containing a portion corresponding to an x-ray absorption filter. The invention also relates to an image processor to process an image formed by x-ray imaging, which contains a portion corresponding to an x-ray absorption filter. The invention further relates to an x-ray examination apparatus comprising an x-ray absorption filter between an x-ray detector and an x-ray source for irradiating an object to form an x-ray image on the x-ray detector.

An image processing method of said kind is known from EP 0309813. According to the disclosure of this document, an X-ray diagnostic installation has an X-ray source, an X-ray image intensifier video-chain and a control circuit connected to the output of the video chain which includes a circuit for image processing. In particular the image is divided into a number of individual regions and mean intensity values are estimated in said regions. A threshold circuit for contour recognition compares the measured mean brightness values of the individual image regions to a brightness threshold and transmits through those measured signals corresponding to darker regions which are referred to as regions of relevance, blanking the other regions referred to as irrelevant regions. The x-ray diagnostic installation also comprises a collimator having movable plates to adjust the size of said x-ray beam and includes means for generating a signal for positioning plates of said collimator based on means for identifying the irrelevant regions.

An other image processing method and an apparatus for detecting colinear edge points and measuring the distance between two edges is already known from WO92/11609. According to the disclosure of said document, in a digital image, the pixels corresponding to edge points are identified. Then a straight line is determined as the line passing through the maximum number of said point. The search for this line is performed by varying test slope values.

An image processing method of said kind is also known from the US patent US 4 670 896; therein are disclosed an x-ray examination apparatus and the use thereof to form on the x-ray detector the x-ray image containing a portion corresponding to the x-ray absorption filter. The cited reference teaches that a substantial difference in image-contrast can occur between parts of an x-ray image formed due to irradiation of the object by x-rays. Brightness differences forming image contrast arise e.g. in peripheral angiography because part of the x-ray beam does not penetrate the object, notably a patient to be examined, and is incident directly on the x-ray detector, or for example in cardio-examination because the object exhibits substantial differences in x-ray absorption, viz. a patient's heart having comparatively high x-ray absorption is surrounded by lung-tissue which is relatively transparent for x-rays. In order to make the dynamic range of the x-ray image coincide as well as possible with the contrast in the region of interest, x-ray absorption filters e.g. having the form of x-ray shutters, are arranged between the x-ray source and the object. The x-ray shutters are positioned to intercept a part of the x-ray beam so as to shield relatively transparent parts of the patients anatomy form the x-rays. The use of such x-ray shutters leads to diminishing an x-ray doses to which a patient is exposed to produce an x-ray image. Moreover, an x-ray dose whereto a person who operates the x-ray examination apparatus is unintentionally exposed is diminished by the use of x-ray shutters. In the x-ray image x-ray shutters appear as regions having extremely high absorption. Consequently, in inverted images, as are often used by radiologists for examination, x-ray shutters appear as bright areas which distract attention from medical information. Furthermore, when a plurality of images made by x-ray irradiation is brought together onto a hard copy, such as an x-ray film, a substantial portion of the area of the x-ray film is wasted when said images contain areas corresponding to x-ray shutters. Thus, making images, in accordance with the cited reference, by irradiating an object by an x-ray beam and employing the x-ray absorption filter arranged between the x-ray source and the x-ray detector has several shortcomings and disadvantages.

An other processing method is known of the patent US-A-4,961,425. This patent describes a system for automatically determining the outline of a selected anatomical feature or region. The outlines are based on intensity contours, where the intensity of the contour is intermediate that within and outside of the feature. A technique for choosing an initial outline is to examine the drop or rise in intensity along a radical direction from a starting point within the region of interest and assign the contour to the location at which the difference in intensity reaches a predetermined value. As this method is to be applied to slices of magnetic resonance data, the optimized initial outline is saved and used for the adjoining slice. The accuracy of the out line is improved using an edge-optimization procedure in which the outline is shifted transversely to the location at which an estimate of the derivative is maximum.

Therefore, it is an object of the invention to provide an image processing method for reducing harmful effects of areas in the image corresponding to the x-ray absorption filter on the quality of the image made by x-irradiation.

This object is achieved by the image processing method according to the invention to process an image formed by x-ray imaging, the image containing a portion corresponding to an x-ray absorption filter, said image processing method comprising :
- determining an edge of said portion,
- modifying the image by selectively processing said portion,
characterized in that the detection of the edge comprises :
a grouping step to select a group of transition-points in the image having a higher brightness towards the centre of the image and a lower brightness towards the periphery of the image, and a curve-fitting step to fit a curve through said group of transition-points, said curve forming a representation of said edge.

Transition-points in the image that lie on an edge of an x-ray shutter-area have high brightness towards the centre of the image and low brightness towards the periphery of the image. For each edge in the image a number of transition-points are determined and grouped into groups of transition-points for respective x-ray shutter-edges. Representations of respective x-ray shutter-edges are obtained by performing a curve-fitting method for each of the groups. The result is one or a number of curves being representative for x-ray shutter-edges in the image.

A further preferred implementation of an image processing method in accordance with the invention is characterised in that the grouping step comprises a region-selection step to select a region of interest extending from a periphery of the image and a division-step to divide said region of interest into sections and a weight-assignment step to assign weights to said transition-points, a weight for a transition-point depending on image features around said transition-point and a weight for a transition-point depending decreasingly on a distance between said transition-point and said periphery.

In an image made by x-irradiation it is known beforehand that x-ray shutter-areas extend from the periphery of the image because the x-ray shutters are inserted in the x-ray beam during x-ray exposure from the outside of the x-ray beam. Consequently, it is effective to carry-out grouping of transition-points in accordance with known topology of the image. A group of transition-points being representative of an x-ray shutter-edge has transition-points located in a region of interest that extends from the periphery of the image. A brightness-transition is a necessary but not a sufficient feature of a point in the image for being on an x-ray shutter-edge. Detection of transition-points is carried-out by dividing a region-of-interest into sections, notably elongated strips. Subsequently, brightness-values are averaged in a direction perpendicular to the longitudinal axis of elongated strips. Then, one or several points having a brightness transition are determined in each of the sections. In each of the sections, the brightness-transition being closest to the periphery of the image is the most likely candidate for being on an x-ray shutter-edge. This is implemented by assigning weight-factors to brightness-transitions found in each of the sections, each weight-factors depending decreasingly on the distance between a brightness-transitions at issue and said periphery of the image.

A further preferred implementation of an image processing method in accordance with the invention is characterised in that the grouping step comprises a first-estimate step to determine a first-estimate for a position of a transition-point in a section and an accurating step to determine a transition-point from said first-estimate.

Processing speed is increased by performing a first estimate for a transition-point on the basis of a component of the image having only low spatial frequency variations of brightness-values. Such a component is obtained by performing a local spatial averaging of brightness-values in the image. After a first estimate for a transition-point has been obtained, the position of said transition-point can be determined more accurately by considering brightness-values of the image in a neighbourhood of the first estimate. Thus, it is avoided to a large extent to search in vain for transition-points in regions of the image where there are no substantial brightness variations.

A further preferred implementation of an image processing method in accordance with the invention is characterised in that the weight-assignment step comprises computation of image roughness, and computation of transition-height, and computation of said weights depending on image roughness and transition height for respective transition-points.

Brightness-transitions that relate to x-ray shutter-edges are distinguished from brightness-transitions that for example relate to anatomical structures by taking image features around these transitions into account. Image roughness is defined as e.g. the statistical variance of brightness-values or e.g. the maximum of the differences of pixel-values of pixels having a predetermined distance in the image between them. It is found empirically that image-roughness is considerably lower in the low-brightness region in an image caused by an x-ray shutter. Also the height of a transition due to an x-ray shutter-edge is higher than a brightness-transition due to anatomical structures. Therefore, selection of brightness-transitions relating to x-ray shutter-edges is made more effective if the weight-factors of the brightness-transitions are made dependent on the above image features. This dependence is such that a brightness-transition having image features indicating an x-ray shutter-edge are given higher values for its associated weight-factor. There can be several transition-points in a section, then transition-points located nearer to the periphery of the image add to image-roughness for transition-points located nearer to the centre of the image. Consequently, a dependence which is decreasing with the distance between the transition-points and the periphery of the image for weight-factors for transition-points within a section is obtained.

A further preferred implementation of an image processing method in accordance with the invention is characterised in that the weight-assignment step comprises computation of first and second spatial derivatives of brightness-values near respective transition-points and employing substantial concurrence of a maximum of said first derivative with a brightness-variation and with a maximum of said second derivative at the peripheral side of said brightness-variation for selecting the position of said brightness-variation as a transition-point and assigning said weight on the basis of said concurrence.

Brightness-transitions due to x-ray shutter edges have a particular image feature that distinguishes x-ray shutter-edges from brightness transitions for example due to anatomical structures or vignetting. It is found that a brightness-transition of an x-ray shutter-edges has a maximum spatial first derivative of the brightness values near the brightness-transition in the image and has also a maximum of the spatial second derivative of the brightness values slightly peripheral to the brightness-transition in the image. This feature is employed in selection of transition-points by assigning a low weight to transition-points not having a substantial value for their maxima of first derivatives and not having a maximum second spatial derivative slightly peripheral to the transition-point.

A further preferred implementation of an image processing method in accordance with the invention is characterised in that the weight-assignment step comprises computation of first spatial derivatives of brightness-values peripheral to respective transition-points, computation of respective average brightness-value of pixels towards the centre to said transition-points, computation of respective ratios of said respective first spatial derivatives and said respective average brightness-values and assigning said weights on the basis of said ratios.

An x-ray shutter-edge gives rise to a brightness-transition in the image stronger than contrast due to an anatomical structure. Another way of distinguishing an x-ray shutter-edge from anatomical structures in the image consists of considering at a transition-point a gradient of brightness-values in a direction towards the centre of the image. A value of such a gradient is subsequently connected to the average brightness-values between said transition-point and a periphery of the image. If said gradient is comparatively strong and said average corresponds to high brightness then said transition-point is comparatively more likely to be on an x-ray shutter-edge. A weight for a transition-point is made dependent on the ratio of said gradient and said average, so that a transition-point is assigned a higher weight when it is likely to be on an x-ray shutter edge.

A further preferred implementation of an image processing method in accordance with the invention is characterised in that the weight assignment step is followed by a point-selection step for selecting a transition-point having the highest weight in a section and retaining only said selected transition-points in said selected group.

Processing speed of the image processing method is increased by retaining as few transition-points as possible. The transition-points that are retained should preferably be as likely as possible to be on an x-ray shutter-edge instead of being representative for anatomical structure. Such transition- points are selected by sorting transition-points of a section on the basis of their respective weights and retaining in each section only the transition-point having the highest weight in its section.

A further preferred implementation of an image processing method in accordance with the invention is characterised in that said curve-fitting step is followed by a reassigning step for reassigning a value to a weight of a transition-point in dependence on a distance between said transition-point and a curve fitted through the group whereto said transition-point belongs, and in that the curve-fitting step is re-iterated employing said re-assigned weight.

When anatomical structures are visible due to scatter radiation in an x-ray shutter-area, transition-points within the x-ray shutter-area may be selected in the point-selection step. The ensuing curve fitting step will then produce a curve to which selected points may have a comparatively large distance. This occurs because anatomical structures have a more complicated structure than approximately straight x-ray shutter-edges. To prevent that image structures that are e.g. due to scatter radiation are mistaken for x-ray shutter-edges, weight-factors of the transition-points are decreased on the basis of said distance. Transition-points which are close to the fitted curve have a higher likelihood to be actually on the edge of an x-ray shutter-area; therefore, weight-factors of transition-points are adapted before performing a next iteration-step in correspondence with said likelihoods. The iteration is performed particularly effectively because an adequate data-set for starting the iteration is chosen as follows. For each section a peak-point is selected among the transition-points in said section. A peak-point is a transition-point that has the smallest average distance between said peak-point and a predetermined number of neighbouring transition-points; empirically it has appeared that the choice of five neighbouring transition-points is very adequate to determine a peak-point. The first estimate for a curve representing an x-ray shutter edge is then taken to be a curve that connects peak-points of respective sections.

A further preferred implementation of an image processing method in accordance with the invention is characterised in that the edge-determination step is followed by an edge-verification step comprising computation of a reliability-parameter for an edge being determined and comparison of said reliability-parameter with a predetermined threshold level.

Even in the cases where no x-ray shutter was employed during irradiation the image processing method in accordance with the invention may produce a curve allegedly identifying an x-ray shutter-edge. In such instances an anatomical structure would mistakenly regarded as an x-ray shutter. To avoid such errors, an edge-verification step is performed. Anatomic structures differ from x-ray shutter-edges in that the latter give rise to comparatively straight curves. A plurality of said straight curves constitutes a, possibly complex, circumference of a section of the x-ray beam that is passed through the x-ray absorption filter. Moreover, if a curve detected in a region of interest extending from one periphery of the image indeed represent an x-ray shutter edge, then most often another x-ray shutter-edge is present in a region of interest extending from an opposite periphery of the image. An average distance of a predetermined number of transition-points generates a quantitative measure of straightness of a curve. A reciprocal of said average distance is employed as a reliability parameter. If said reliability parameter has a value below a predetermined threshold-value then it is decided that the detected curve does not for certain relate for certain to an x-ray shutter-edge and the step of reducing the area peripheral to said curve consists of leaving the pixel-values in said area unaltered. If the reliability exceeds said threshold-value then a presence of an x-ray shutter-edge in an opposite region-of-interest is verified. If no such opposite x-ray shutter is found then the fitted curve is discarded and again the step of reducing the area peripheral to said curve consists of leaving the pixel-values in said area unaltered.

A further preferred implementation of an image processing method according to the invention is characterized in that for a plurality of transition-points said reliability parameter is computed and in a situation where comparison of said reliability parameter to said predetermined reference level indicates the presence of said portion corresponding to an x-ray absorption filter for less transition-points than a predetermined reference number the edge-verification step is supplemented by comparison of pixel-values of collections of pixels on different sides of the curve.

When the number of transitions point retained for fitting the curve is less than a predetermined reference number then an ambiguous situation with regard to deciding on the presence of an x-ray shutter arises. Then according to the invention the average intensity of collection of pixels on either sides of the curve are compared. Should these average intensities differ more than a predetermined reference level than it is decided that an x-ray shutter is present because a high mean intensity on the side of the curve towards the centre of the image and a low mean intensity on the side of the curve toward the periphery of the image is compatible with the presence of an x-ray shutter. The predetermined reference number may be set to a very high value, for instance equal to the total number of pixels of the image, in order to achieve that supplementing the edge-verification step is performed in almost any situation.

To reduce the computational burden said average intensities may be calculated for comparatively small regions in the image, for example two vertical line segments an inner one with respect to the calculated curve lying towards the centre of the image and the other, outer one, lying towards the periphery of the image.

To improve the reliability of the edge verification the maximum value Lmax of the second spatial derivative (Laplacian) of the pixel-values on the outer segment is determined. If this maximum value is found to be large, it indicates the presence of a anatomical structure towards the periphery of the accurate transition-point at issue and hence the likelihood of the actual presence of an x-ray shutter is reduced.

A further preferred implementation of an image processing method according to the invention characterized in that plurality of transition-points said reliability parameter is computed and in a situation where comparison of said reliability parameter to said predetermined reference level indicates the presence of said portion corresponding to an x-ray absorption filter for less transition-points than a predetermined reference number the edge-verification step is supplemented by determining a magnitude of a first spatial derivatives of brightness-values of pixels in the image between the curve and the periphery of the image, said derivative being negative in the direction from the curve to the periphery of the image and comparing said magnitude to a predetermined reference value.

When the number of transitions point retained for fitting the curve is less than a predetermined reference number then an ambiguous situation with regard to deciding on the presence of an x-ray shutter arises. Then according to the invention the gradient of the intensity for collection of pixels on either sides of the curve are compared to each other. As intensities, and thus pixel values, hardy ever decrease strongly within an x-ray shutter-area in a direction towards a periphery of the image, considering the most negative slope constitutes a reliable test for the presence of an x-ray shutter in an ambiguous situation. Strongly decreasing pixel-values peripheral from the computed curve therefore indicate that the computed curve does not represent an x-ray shutter-edge.

A processed image that does not show harmful effects such as distracting features in the portion corresponding to the x-ray absorption filter appearing in the image as x-ray shutter-areas is obtained in accordance with the image processing method of the invention since portions of the x-ray image corresponding to an x-ray absorption filter i.e. for instance x-ray shutter-areas are modified for example in that these are removed from the image. Then in the processed image the portion corresponding to an x-ray shutter area is absent. Pixel-values in the x-ray shutter-area may also be set to a predetermined grey-level or are given a non-distracting uniform colour-value. Therefore, in the processed image the portion that corresponds to x-ray shutter-areas is presented as an area having a non-distracting grey-tone or inconspicuous colour and consequently the processed image only contains image information that is relevant.

It is a further object of the invention to provide an image processing method for identifying the portion corresponding to an x-ray absorption filter before selectively processing the image portion which corresponds to the x-ray absorption filter.

A preferred implementation of an image processing method in accordance with the invention is characterised in that the processing comprises determining an edge of the portion for selecting said portion as a part of the image between the edge and a periphery of the image.

Sections of the x-ray beam that are absorbed by the x-ray absorption filter may have a complicated shape corresponding to complicated anatomical structures of a patient to be examined. Therefore it is difficult and often quite impossible to know beforehand or predict the location of an area which forms the portion of the image corresponding to said filter, i.e. an area of the image in which x-rays were absorbed due to the x-ray shutters. In accordance with the invention an image processing method is provided that can be performed independently of the x-ray examination apparatus and/or adjustments thereof that is employed to irradiate the object. Furthermore, it is often desired to process the image separately from the x-ray examination. Any information regarding x-ray shutter-positions and/or adjustment will then be lost.

In images made by x-irradiation of an object one or several x-ray shutters are employed for avoiding overexposure in parts having low absorption and being irrelevant from a medical point of view. Such x-ray shutters are without exception inserted into the x-ray beam from the edge of the x-ray beam towards the centre thereof. Consequently, when the location of the edge of an x-ray shutter is determined it is certain that the entire region of the image from said edge towards the periphery of the image is an x-ray shutter-area, i.e. an area in the image where x-radiation has been absorbed by the absorption filters.

When locations of edges of x-ray shutter-areas have been determined, a processed image wherein x-ray shutter-areas are reduced in that said x-ray shutter-areas are converted into a simpler form by deleting image information of x-ray shutter-areas or by setting pixel-values in areas peripheral to the x-ray shutter-edges to a predetermined grey-level or a non-distracting colour-value. A processed image that does not have distracting features in x-ray shutter-areas is obtained in accordance with the image processing method of the invention since x-ray shutter-areas are removed from the image and/or pixel-values in the shutter-area are set to a predetermined grey-level or colour-value. Therefore, in the processed image regions that correspond to x-ray shutter-areas are presented as parts having a non-distracting grey-tone or inconspicuous colour and consequently the processed image only contains image information that is relevant.

A preferred implementation of an image processing method in accordance with the invention is characterised in that the processing comprises removing at least a part of said portion from the image.

A particular advantage of removing portions of a portion of forming an x-ray shutter-area is achieved when a collection of processed images are brought together on a hard-copy, e.g. an x-ray film. As irrelevant x-ray shutter-areas are discarded, less space on the x-ray film is required, so that on the same area of the x-ray film more images can be collected.

A further preferred implementation of an image processing method according to the invention is characterized in that the processing comprises assigning substantially uniform values to pixels in the portion in the image.

Distracting portions in the image are avoided in that x-ray shutter-areas which do not contain medical relevant information are converted into portions having a uniform grey-tone or an inconspicuous colour. Then the processed image is suitable to be considered by a physician who may comfortably focus his/her attention to the medical information contained in the image without being distracted by the x-ray shutter-areas.

It is also an object of the invention to provide an image processor to process an image formed by x-ray imaging, which contains a portion corresponding to an x-ray absorption filter device and which is arranged to carry out the image processing method of the invention. This is achieved by an image processor according to claim 15 wherein the processor comprises a device for determining an edge of the portion according to the above described method and a device for modifying the image by selectively processing the portion.

Moreover, it is an object of the invention to provide an x-ray examination apparatus comprising an x-ray absorption filter between an x-ray detector and an x-ray source for irradiating an object to form an x-ray image on the x-ray detector and which is arranged to carry out the image processing method of the invention. This is achieved by an x-ray examination apparatus according to the invention, characterized in that the apparatus comprises an image processor according to Claim 5 and having an input port coupled to the x-ray detector.

These and other aspects of the invention will become apparent from and will be elucidated with reference to the implementations and the embodiments described hereinafter and with respect to the accompanying drawings wherein :
Figure 1 shows a diagrammatic representation of an implementation of an image processing method in accordance with the invention;
Figures 2a-c shows an exemplary variation of brightness around an x-ray shutter-edge, and first and second spatial derivatives of said variation;
Figure 3 shows a diagram of a division into sub-regions in accordance with the image processing method in accordance with the invention of a region of interest;
Figure 4a shows a diagrammatic illustration of detection of x-ray shutter-edges in an image made by x-ray exposure;
Figure 4b shows a result of an image processing method in accordance with the invention having been applied to the image shown in Figure 3b;
Figure 5 shows a schematic representation of an x-ray examination apparatus comprising a device for performing an image processing method in accordance with the invention ;
Figure 6 shows a diagrammatic representation of a further implementation of an image processing method in accordance with the invention;

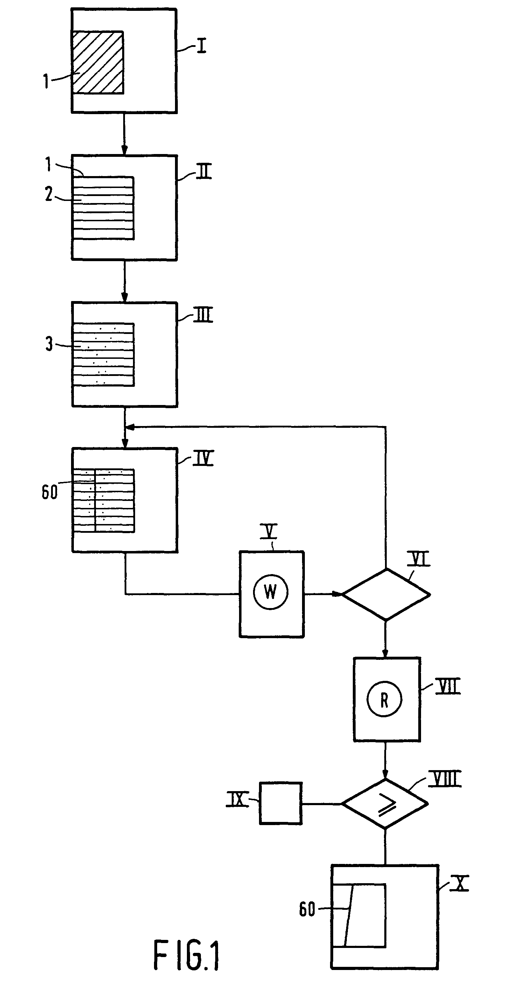

Figure 1 shows a diagrammatic representation of an image processing method in accordance with the invention. In block I a region of interest 1 is selected wherein an x-ray shutter-edge is expected. In block II said region of interest 1 is divided into sections 2. On the basis of brightness values in each of the sections 2, transition-points are determined, one of them being indicated by the reference numeral 3. Furthermore, weights are assigned to the transition-points and a curve is fitted through the transition-points where use is made of the weights for performing the curve fitting so as to obtain a fair representation of an x-ray shutter-edge. In block IV a fitted curve 60 is shown. Once a curve has been determined, further accuracy is achieved by computing weights for each of the transition-points where for a transition-point a weight is computed that depends on the distance between the fitted curve and said transition-point. Reassigning weights is performed in block V and the curve-fitting process is re-iterated under control of a comparison step that is represented by block VI. When this iteration has converged, reliability of the converged curve is computed in block VII on the basis of the weight. In this way, because x-ray shutter-edges have transition-points that have comparatively high weights whereas anatomical structures have transition-points with lower weights, it is avoided to a large extent that anatomical structures are mistaken for an x-ray shutter-edge. To that end a computed reliability is compared in a comparison step represented by block VIII with a predetermined threshold level that is provided from a memory means IX. In a final step shown in block X, image information of the x-ray shutter-area, being the area peripheral to the fitted curve, is deleted. The deletion may consist of setting all pixel-values on the x-ray shutter-area to a grey-level or a non-distracting colour-value that may be either predetermined or can be selected. Thus, said x-ray shutter-area is reduced in that image information of the x-ray shutter-areas is deleted. As an alternative the x-ray shutter-area is reduced in that said x-ray shutter-area is discarded from the image.

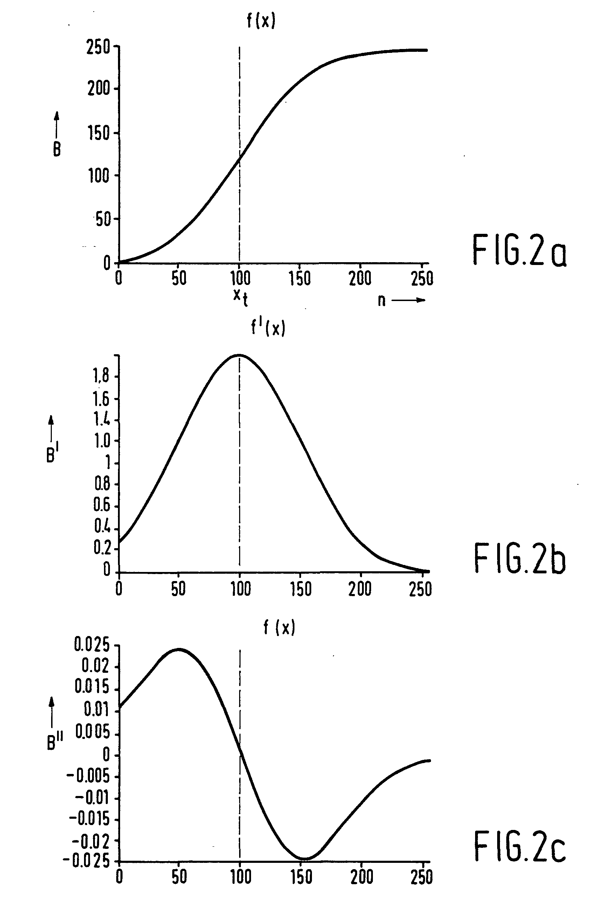

Figures 2a-c shows an exemplary variation of brightness around an x-ray shutter-edge, and first and second spatial derivatives of said variation. Figure 2a shows dependence of brightness-values as a function of position along a line in an image made by x-ray exposure. The coordinate x increases from the periphery of the image towards the centre of the image. As Figure 2a shows the brightness-value B is very low near the periphery of the image owing to the presence of an x-ray shutter. There is a transition to high brightness-values around the position xt which is representative for the edge of an x-ray shutter. Figure 2b shows the first spatial derivative B'(x) in the direction of said line of the brightness variations shown in Figure 2a. Notably, at the x-ray shutter-edge xt the derivative B' has a maximum, indicating the brightness variations being the most pronounced at the x-ray shutter-edge. Figure 2c shows the second derivative B"(x) in the direction of said line. As appears from Figure 2c, the second derivative has a maximum close to the position xt, where the first derivative has a maximum. In relation to the maximum of the first derivative, the maximum of the second derivative has a position that is towards the periphery of the image. The feature that the maximum of the first derivative at the side facing the periphery of the image is accompanied by a maximum of the second derivative, discriminates a brightness-transition having low brightness on the peripheral side and having high brightness on the side towards the centre from a brightness-change having low brightness on the peripheral side and gradually increasing brightness towards the centre; such a brightness-change in practical situations often arises due to vignetting in the image.

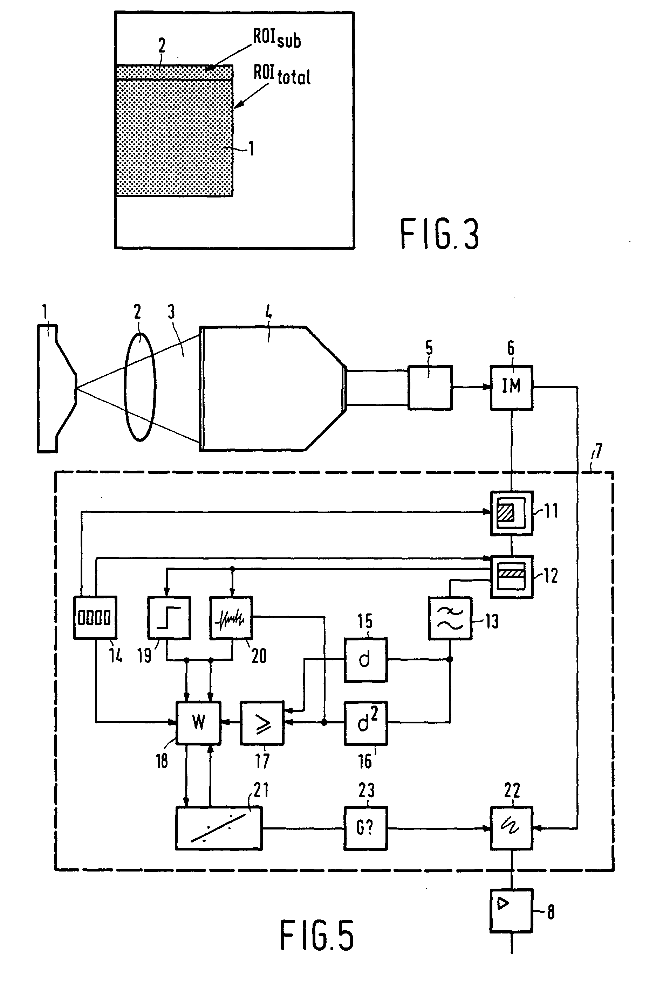

Figure 3 shows a diagram of a division into sub-regions of a region of interest in accordance with the image processing method in according to the invention .

To search for an x-ray shutter-edge of an x-ray shutter located in the left part of the image first a region of interest ROItotal 1 is selected as is indicated in Figure 3. The region ROItotal extends over half the vertical dimension of the image and from the left edge of to the middle of the image in the horizontal direction. In this manner, artifacts in the corners are left out of consideration and risk of conflict with other x-ray shutters being present is diminished. Other x-ray shutters, such as right-vertical, top-horizontal or bottom-horizontal can be detected in accordance with the invention by choosing regions replacing vertical and horizontal and top and bottom. Subsequently, the region ROItotal, which consists e.g. of 256×256 pixels is divided into elongated strips ROIsub, one of them being shown of e.g. 256×32 pixels. For each of the strips pixel values are averaged in the vertical direction, i.e. to each of the 256 positions in the longitudinal direction of the strip currently being processed the average of the 32 pixel-values having the same horizontal position is assigned. This yields a dependence of (average) brightness along the elongated direction of the current strip, as is depicted e.g. in Figure 2a.

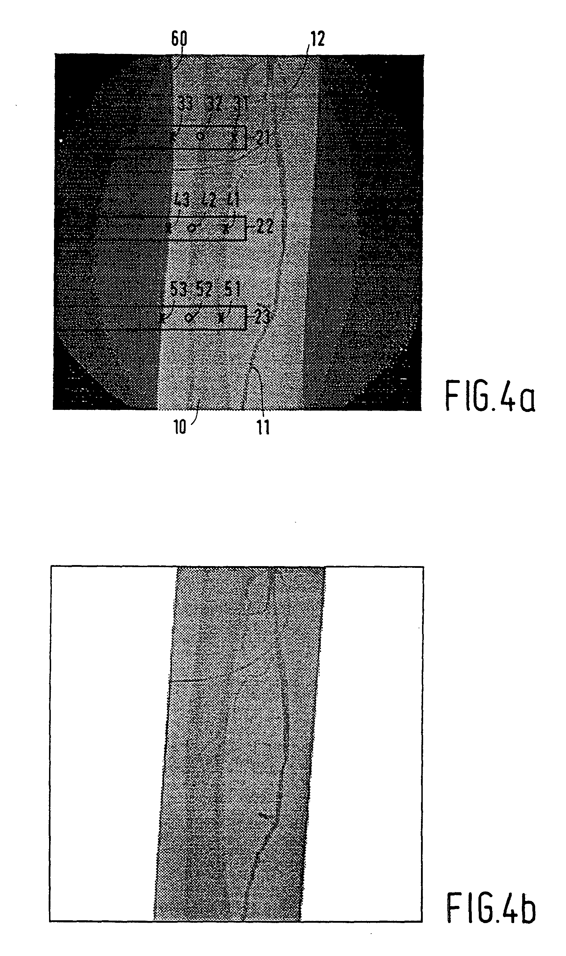

Figure 4a shows a diagrammatic illustration of detection of x-ray shutter-edges in an image made by x-ray exposure. Figure 4a shows an image made by x-ray exposure featuring various anatomical structures such as a bone 10 and a artery 11 and a catheter 12. Furthermore, several elongated strips 21, 22 and 23, similar to the strips 2 in Figure 3 are indicated. In each of the strips several brightness transitions occur. These brightness-transitions originate from either anatomical structures or from an x-ray shutter. E.g. in strip 21, at position 31 brightness changes from high to low when proceeding towards the periphery of the image, at position 32 the brightness B increases to a higher value and finally at position 33 brightness again drops to a very low value. Consequently, both brightness transitions at 31 and 33 are accompanied by respective maxima of the spatial derivatives B' and B", as discussed with reference to Figures 2a-c. On the other hand the brightness transition at position 32 is accompanied by a minimum of B', so that only the transitions at 31 and 33, respectively are considered candidates for a sought x-ray shutter-position. Because the transition at position 31 is accompanied by another transition being more peripheral, viz. the transition at position 33, to position 31 a smaller weight-factor is assigned and to the position 33 a large weight-factor is assigned.

Similarly, two further strips 22 and 23 are indicated, each of them comprising several brightness transitions at positions 41, 42, 43 and 51, 52, 53, respectively. Of these, the transitions at 43 and 53 are assigned a large weight-factor value. By a known curve-fitting method a curve 60 is drawn through the group of transition-points formed by (31, 33, 41, 43, 51, 53). The curve 60 forms a fair estimate of the x-ray shutter-edge of a left x-ray shutter.

The accuracy of the fitted curve which is representative for an x-ray shutter-edge is increased by iterating. After an estimate is obtained, weight-factors are re-assigned to the positions that were used for obtaining the current fitted curve. The process is started by determining peak-points in each of the sections and choosing a curve that connects said peak-points as a first estimate for a curve representing an x-ray shutter-edge at issue. A peak-point is a transition-point such that the average distance between a predetermined number of neighbouring transition-points and said peak-point is the smallest; empirically it has appeared that the choice of five neighbouring transition-points is very adequate to determine a peak-point. When re-assigning weight-factors, distances between said positions and the current fitted curve are taken into account such that positions that are close to the current fitted curve are given higher weight-factors than position that are more distant from the current fitted curve, for example by means of a Gaussian weight function. Then, on the basis of new values of weight-factors being assigned, again positions being most probable candidates for lying on an x-ray shutter-edge are selected and curve fitting is carried-out employing said new weight-factor values.

Still further refinement of the reliability of the image processing method in accordance with the invention is achieved by considering image features around a transition-point, i.e. a presumed x-ray shutter-edge. A first feature parameter of a brightness-transition is the height of the transition which is determined by the difference between an averaged brightness values at the side of the transition towards the periphery of the image and an averaged brightness at the side of the transition towards the centre of the image. The averaging is e.g. performed over a predetermined number of pixels at a relevant side of a transition at issue.

A second feature parameter is the roughness of the image at the peripheral side of a brightness-transition at issue. In practical situations, x-ray shutters appear distinct from anatomical structures in that within an x-ray shutter-area there is very little structure; this is quantified by image roughness which is defined as the statistical variance of brightness-values in an area of the image at issue. An attractive alternative for quantifying roughness is obtained on the basis of second spatial derivatives of brightness which are interpreted as the difference between a local brightness-value and a running average built-up by three neighbouring pixel-positions in total. Then roughness is defined quantitatively as a reciprocal of an average over the modulus of second spatial derivatives in an area of the image at issue. Low roughness values then correspond to an area of the image having little image structure, thus having a high probability of being an x-ray shutter-area. Further an image processing method in accordance with the invention is now refined in that weight-factors that are assigned to positions that are recognised as candidates for being on an x-ray shutter-edge are made increasing as a function of the height of the transition and are made decreasing as a function of the roughness in the area of the image peripheral of the candidate at issue.

After all x-ray shutter-edges in the image have been determined, image-information in the areas of the image peripheral to any of the curves corresponding to x-ray shutter-edges is deleted from the image and as a result a processed image comprising only relevant medical information is obtained. In particular, in areas corresponding to x-ray shutters as determined according to the invention, all pixels are set to the same value; in this manner x-ray shutter-areas are blanked. Figure 4b shows a processed image obtained from the image shown in Figure 4a.

The processed image has as an advantage that it does not include parts that are distracting when examining the image. Furthermore, when a hard-copy e.g. on film of a plurality of images having been processed according to the invention is made, an advantage is achieved in that more economic use is made of hard-copy material.

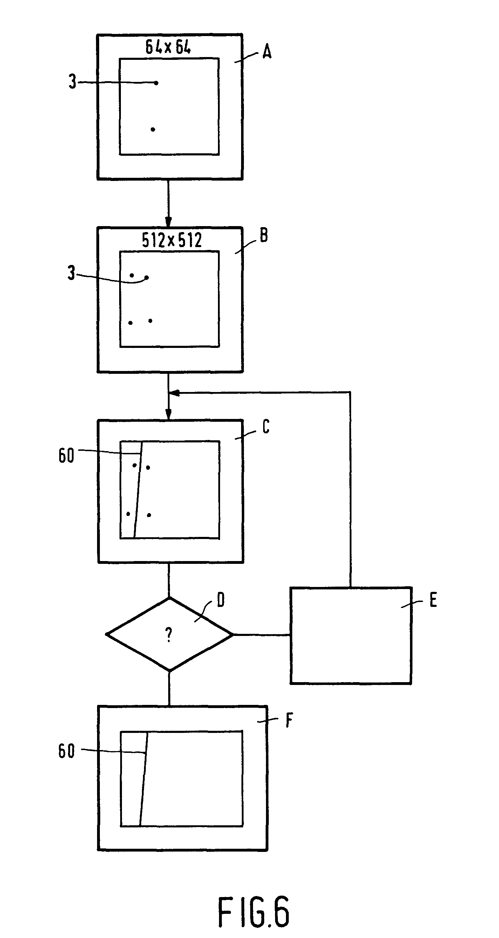

Figure 5 shows a schematic representation of an x-ray examination apparatus comprising a device for performing the image processing method in accordance with the invention. An x-ray source 1 is provided for irradiating an object 2 so that an image carrying x-ray beam is formed. By means of an x-ray detector 4 and an image read-out device 5 an image made by x-ray exposure is formed an supplied to an image-memory 6. An image processor 7 is employed for converting the image stored in the image-memory 6 into a processed image from which x-ray shutter-areas are deleted. The processed image is supplied to an image buffer-circuit 8 for further processing such as display on a monitor screen, formation of a hard-copy or to be archived.

The image processor 7 will now be described in more detail. A selector 11 selects a region of interest ROItotal wherein an x-ray shutter-edge is to be detected. By a divider 12 the region of interest is divided into elongated strips ROIsub. The selector 11 and the divider 12 are controlled by an iterator 14 for subsequently selecting said elongated strips for determining an x-ray shutter-edge and for subsequently selecting regions of interest for determining various x-ray shutter-edges. Brightness-values of pixels in an elongated strip are averaged along the transverse direction of the elongated strip at issue so that a one-dimensional brightness distribution being representative for image information along the longitudinal direction of the elongated strip, is formed by an averager 13. First and second derivatives of the one-dimensional brightness distribution are computed by differentiators 15 and 16, respectively. From the output of the differentiators candidate-positions for x-ray shutter-edge positions are determined by a comparator 17. To candidate x-ray shutter-edge positions weight-factors are assigned by way of a weighting-means 18. Said weight-factors are computed by the weighting means 18. To that end the height of a brightness-transition at a positions at issue is computed by subtracter 19; the roughness associated to the brightness-transition at issue is computed by an evaluator 20. Through the candidate-x-ray shutter positions in successive elongated strips and using their associated weight-factors candidate x-ray shutter positions are selected and a curve is fitted by a curve-fitter 21. In addition to producing a curve that fits the selected positions, the curve-fitter computes updated weight-factors depending on the distances between respective selected positions and the fitted curve. Under control of the iterator 14, selection of candidate x-ray shutter-positions and subsequent curve-fitting is repeated so as to obtain a more reliable result for a curve being a fair approximation of an x-ray shutter-edge. The curve found after iteration has converged is supplied to a verification-means 22 for to verify that said curve is indeed representative of an x-ray shutter-edge. The verification-means comprises a computation unit for calculating an average distance to a curve at issue of a predetermined number of transition-points. In particular five transition-points are chosen that have the smallest distances to the curve at issue. Moreover, the verification means comprises detection means for ascertaining that for a curve at issue, an x-ray shutter-edge is present in an opposite region of interest in the image. Subsequent to verification of approximating curves for various x-ray shutter-edges being present in the image stored in the image-memory 6, parts of the image being peripheral to any said x-ray shutter edges are deleted by an eraser 23 and the processed image obtained in this manner is supplied to the image buffer-circuit 8.

It is noted that in an x-ray examination apparatus in accordance with the invention, the functions of the image processor may be performed by a suitably programmed computer or by a special purpose processor having circuit-means that are arranged to perform said functions.

Figure 6 shows a diagrammatic representation of a further implementation of an image processing method in accordance with the invention. In block A a rough detection of transition-points is performed. In this phase of the image processing method the full image having, for example, 512×512 pixels is subsampled down, for example, to 64×64 pixels by taking one point out of eight in the horizontal and vertical directions. This considerably reduces the computational burden at the cost of a lesser accuracy in localisation of the transition-points. Accuracy will be recovered in the sequel of the image processing method, viz. in block B. The following computational steps are performed. The subsampled image is smoothed using a straight vertical kernel. This is done by adding to each pixel-value the pixel-value of pixels in the neighbourhood in said sub-sampled image. In this way, sharp horizontal transitions are retained in this way while vertical transitions are smoothed out.

Subsequently, nine rows are selected as sections, out of the 64 rows of the subsampled image. A systematic search for transition-points that lie on an x-ray shutter-edge is then performed. To that end, in each row the average pixel-value of pixels located between a transition-point and a periphery of the image is estimated by taking the sum of intensities of four pixels located between the pixel for which said average is to be computed and the periphery of the image. Next, the component in a direction orthogonal to a periphery of the image of a gradient of pixel-values is evaluated by computing differences between consecutive points in said section.

In block B accurate localisation of transition-points is represented. From the phase performed in block A, nine roughly localised transition-points are provided. The phase of block B aims at increasing accuracy of localisation of transition-points in the 512×512 image. A local search around each of said nine transition-points is performed within the same section for a neighbourhood consisting of, for example, 17 pixels, viz. eight on either side of a transition-point at issue. The local search comprises smoothing in a direction parallel to said periphery of the image by adding pixel-values that neighbour the transition-point at issue in a direction parallel to said periphery. Subsequently, the sum is computed of four pixel-values of four pixels located between said periphery and the transition-point at issue, said sum being representative for an average brightness towards said periphery. A gradient in a direction orthogonal to said periphery is computed by calculating differences of pixel-values of the transition-point at issue and two of its neighbours that are located between said transition-point and the centre of the image. Next, the ratio of said gradient and said average is computed for each transition-point in each of the sections. Accurate transition-points having a comparatively high probability for being on as x-ray shutter-edge are selected as those transition-points having a value for said ratio being the highest in said neighbourhood of 17 pixels.

Block C represents the fitting of a curve, notably the best fit straight line through the accurate transition-points that are provided from the phase of block B. Accurate transition-points which are comparatively far from said straight line are considered as outliers and are rejected. Alteratively, transition points can be weighted according to the distance to the curve, for example by a Gaussian weighting function. The computational procedure for the phase of block C is iterative; it represents a compromise between accuracy and computation time optimisation. The estimation of the x-ray shutter edge position in the image and angle with a predetermined axis of the image is then refined by determining the best fit straight line to the most likely accurate transition-points. For the following of the discussion, said predetermined axis will be chosen to be a vertical axis of the image, although the implementation is not at all restricted to this choice. The pixels of the image are arranged as a square matrix of columns and rows. The x-ray shutter-edge is first assumed to be vertical, its column number is taken to be that of the accurate transition-point with the largest number of substantially vertically aligned accurate transition-points. Each of the accurate transition-points is assigned a likelihood score. Let Δx represent the maximum change in column number of a said accurate transition-points to account for a maximum angle departure of, for example, ±5° from a vertical edge. The likelihood score of any one of the accurate transition-points is calculated by adding up the number of accurate transition-points with column numbers differing by less than Δx plus those with column number differing by less than Δx/2 plus those with column numbers differing by less than Δx/4. The first guess for the position of the x-ray shutter-edge is taken to be a vertical line at the column number of the accurate transition-point having the highest likelihood score. In practice Δx is chosen to be a width of eight pixels.

To proceed further with a low computational burden and to decrease required computation time, accurate transition-points which lie comparatively far away from said guessed x-ray shutter edge position are rejected, for example according to the following test-rules. If four or more accurate transition-points are within a distance of Δx/4 from said guessed x-ray shutter-edge position then accurate transition-points having a distance larger than Δx/2 are rejected. If this test-rule does not apply, the test-rule is repeated after replacing the value of Δx by 2Δx, If neither of these test-rules apply, i.e. less than four accurate transition-points within a distance of Δx from said guessed x-ray shutter-edge position, it is concluded that no x-ray shutter is present.

The accurate transition-points that have been retained after applying said test-rules are employed to more accurately localise the x-ray shutter-edge position in an iterative way under the control of an iteration control indicated as block E. The initial guess of the x-ray shutter-edge is a vertical line at the column number having the highest likelihood. For each of the retained accurate transition-points respective normal distances to said vertical line are computed and Gaussian weights depending on said normal distances are assigned such that the extreme points for an x-ray shutter having a tilt of 5° with the vertical axis are given weights of ½. The function of these weights is to diminish the influence of those retained accurate transition-points which are too poorly aligned with respect to others. A conventional least mean square regression is employed to determine a line that is representative for a new estimate of an x-ray shutter-edge position. In the iteration process, normal distances of retained accurate transition-points are recalculated for the new estimate for the x-ray shutter-edge position. Subsequently, new Gaussian weights are assigned where a Gaussian distribution with a smaller width is used. Following that, a new best fit straight line is determined, thus improving the estimate for the position of the x-ray shutter edge.

After a line that represents an x-ray shutter edge position has been provided by the phase of blocks C and E by iteration a decision-taking phase follows as is represented by block D. After the iteration has converged the root mean square distance Δp of the retained accurate transition-points is calculated. The following situation may then occur. If Δp is larger than 2.6 pixels it is decided that no x-ray shutter is present. The image processing method is then terminated. The value of 2.6 pixels has been determined empirically and may be different in other implementations or applications. If all nine accurate transition-points are retained and Δp is less than 1.2 pixels, it is decided that there is an x-ray shutter and the image processing method proceeds by reducing the area 61 that is peripheral to the line provided by the phase of block D. If only eight accurate transition-points are retained and Δp is less than 0.8 pixels it is also decided that there is an x-ray shutter and subsequently the x-ray shutter-area is reduced in block E. If none of the above mentioned situations occur the presence of an x-ray shutter is ambiguous and three further tests are performed in block D.

If such an ambiguous situations occurs, there are provided from previous phases a set of accurate transition-points. To save computation time further tests are carried-out using the 64×64 subsampled image. The mean pixel-value along two vertical line segments an inner one lying towards the centre of the image and the other, outer one, lying towards said periphery are computed. the common length of these two line segments is taken to be slightly smaller than an estimated length of an x-ray shutter and to be compatible with the rounded shape of the x-ray image intensifier. A high mean pixel-value on the inner-segment and a low value on the outer-segment is compatible with the presence of an x-ray shutter. The quantity computed in this test is the ratio Rrl of said two mean pixel-values and that quantity is compared with a predetermined reference value to decide on the presence of an x-ray shutter.

As second test, the maximum value Lmax of the second spatial derivative (Laplacian) of the pixel-values on the outer segment. If this maximum value is found to be large, it indicates the presence of a anatomical structure towards the periphery of the accurate transition-point at issue and hence the likelihood of the presence of an x-ray shutter is reduced. A reliable criterion is a linear combination M of the ratio Rrl and the maximum value Lmax. The coefficients involved in this liner combination are those optimising the statistical recognition results on the part of the image used for training the algorithm. The linear combination M is compared to a predetermined reference value so as to decide on the presence of an x-ray shutter.

A final independent test that is used in an ambiguous situation also aims at rejecting false x-ray shutter detections. In this final independent test the maximum negative slope on a line orthogonal to said periphery of an image is computed between an accurate transition-point at issue and said periphery. If this maximum negative slope is larger than an predetermined reference value it is decided that no x-ray shutter is present. As pixel values hardy ever decrease strongly in a direction of an x-ray shutter, considering said maximum negative slope constitutes a reliable test for the presence of an x-ray shutter in an ambiguous situation. Strongly decreasing pixel-values peripheral from the computed curve therefore indicate that the computed curve does not represent an x-ray shutter-edge.

## Claims

1. An image processing method to process an image formed by x-ray imaging, the image containing a portion corresponding to an x-ray absorption filter, said image processing method comprising :
determining an edge of said portion,
modifying the image by selectively processing said portion,
**characterized in that** the detection of the edge comprises :
a grouping step to select a group of transition-points (3) in the image having a higher brightness towards the center of the image and a lower brightness towards the periphery of the image, said transition-points being assigned weights and a weight for a transition-point depending decreasingly on a distance between said transition-point and the image periphery and
a curve-fitting step (21) to fit a curve (60) through said group of transition-points, said curve forming a representation of said edge.

2. An image processing method as claimed in Claim 1, **characterized in that** the grouping step comprises :
a region-selection step (11) to select a region of interest (1) extending from a periphery of the image and
a division-step (12) to divide said region of interest (1) into sections (2) and a weight-assignment step (18) to assign the weights to said transition-points (3), a weight for a transition-point depending on image features around said transition-point.

3. An image processing method as claimed in claim 1 or 2, **characterized in that** the grouping step comprises a first-estimate step to determine a first-estimate for a position of a transition-point in a section and an accurating step to determine a transition-point from said first-estimate.

4. An image processing method as claimed in Claim 2 or 3, **characterized in that** the weight-assignment step comprises :
computation (20) of image roughness, and
computation (19) of transition-height, and
computation (18) of said weights depending on image roughness and transition height for respective transition-points.

5. An image processing method as claimed in any one of Claims 1 to 4, **characterized in that** the weight-assignment step comprises :
computation (15,16) of first and second spatial derivatives of brightness-values near respective transition-points and
employing substantial concurrence of a maximum of said first derivative with a brightness-variation and with a maximum of said second derivative at the peripheral side of said brightness-variation for selecting the position of said brightness-variation as a transition-point and assigning said weight on the basis of said concurrence.

6. An image processing method as claimed in any one of Claims 1 to 4, **characterized in that** the weight-assignment step comprises :
computation of first spatial derivatives of brightness-values peripheral to respective transition-points, computation (13) of respective average brightness-value of pixels towards the center to said transition-points, computation of respective ratios of said respective first spatial derivatives and said respective average brightness-values and assigning said weights on the basis of said ratios.

7. An image processing method as claimed in any one of Claims 1 to 6, **characterized in that** the weight assignment step is followed by a point-selection step for selecting a transition-point having the highest weight in a section and retaining only said selected transition-points in said selected group.

8. An image processing method as claimed in any one of Claims 1 to 7, **characterized in that** the curve-fitting step (21) is followed by a reassigning step for reassigning a value to a weight of a transition-point in dependence on a distance between said transition-point and a curve fitted through the group whereto said transition-point belongs, and **in that** the curve-fitting step is re-iterated employing said re-assigned weight.

9. An image processing method as claimed in any one of Claims 1 to 8, **characterized in that** determining the edge (60) is followed by an edge-verification step (22) comprising computation of a reliability-parameter for an edge being determined and comparison of said reliability-parameter with a predetermined threshold level.

10. An image processing method as claimed in claim 9, **characterized in that** for a plurality of transition-points said reliability parameter is computed and in a situation where comparison of said reliability parameter to said predetermined reference level indicates the presence of said portion corresponding to an x-ray absorption filter for less transition-points than a predetermined reference number the edge-verification step is supplemented by comparison of pixel-values of collections of pixels on different sides of the curve.

11. An image processing method as claimed in Claim 10, **characterized in that** plurality of transition-points said reliability parameter is computed and in a situation where comparison of said reliability parameter to said predetermined reference level indicated the presence of said portion corresponding to an x-ray absorption filter for less transition-points than a predetermined reference number the edge-verification step is supplemented by determining a magnitude of a first spatial derivatives of brightness-values of pixels in the image between the curve and the periphery of the image, said derivative being negative in the direction from the curve to the periphery of the image and comparing said magnitude to a predetermined reference value.

12. An image processing method as claimed in one of Claims 1 to 11, **characterized in that** the processing comprises :
selecting the portion corresponding to the X-ray absorption filter as a part of the image between the edge and a periphery of the image.

13. An image processing method as claimed in Claim 12, **characterized in that** the processing comprises :
removing at least a part of said portion from the image.

14. An image processing method as claimed in Claim 12, **characterized in that** the processing comprises :
assigning substantially uniform values to pixels in said portion in the image.

15. An image processor to process an image formed by x-ray imaging, which contains a portion corresponding to an x-ray absorption filter, **characterized in that** the processor comprises :
a device for determining an edge of the portion according a method as claimed in one of Claims 1 to 11,
and a device for modifying the image by selectively processing the portion.

16. An x-ray examination apparatus comprising an x-ray absorption filter between an x-ray detector and an x-ray source for irradiating an object to form an x-ray image on the x-ray detector, **characterized in that** the apparatus comprises an image processor according to Claim 15 and having an input port coupled to the x-ray detector.

## Patentansprüche

1. Bildverarbeitungsverfahren zum Verarbeiten eines durch Röntgenbestrahlung gewonnenen Bildes, wobei das Bild einen Teilbereich enthält, der einem Röntgenabsorptionsfilter entspricht und das genannte Bildverarbeitungsverfahren folgendes umfasst:
Bestimmen eines Randes des genannten Teilbereichs,
Verändern des Bildes durch selektives Verarbeiten des genannten Teilbereichs,
**dadurch gekennzeichnet, dass** die Erfassung des Randes folgende Schritte umfasst:
einen Gruppenbildungsschritt zum Auswählen einer Gruppe von Übergangspunkten (3) in dem Bild, die eine größere Helligkeit zur Mitte des Bildes hin und eine geringere Helligkeit zum Rand des Bildes hin aufweisen, wobei den genannten Übergangspunkten Gewichtungen zugeordnet werden und eine Gewichtung für einen Übergangspunkt in ständig abnehmendem Maße von einem Abstand zwischen dem genannten Übergangspunkt und dem Rand des Bildes abhängt, und
einen Kurvenanpassungsschritt (21) zum Anpassen einer Kurve (60) durch die genannte Gruppe von Übergangspunkten, wobei die Kurve eine Darstellung des genannten Randes bildet.

2. Bildverarbeitungsverfahren nach Anspruch 1, **dadurch gekennzeichnet, dass** der Schritt der Gruppenbildung folgendes umfasst:
einen Schritt der Auswahl (11) eines relevanten Bereichs (1), der sich vom Rand des Bildes ausdehnt und
einen Schritt der Aufteilung (12) des genannten relevanten Bereichs (1) in Teilbereiche (2) und einen Schritt der Zuordnung von Gewichtungen (18) zu den genannten Übergangspunkten (3), wobei eine Gewichtung für einen Übergangspunkt von den Bildmerkmalen um den genannten Übergangspunkt herum abhängt.

3. Bildverarbeitungsverfahren nach Anspruch 1 oder 2, **dadurch gekennzeichnet, dass** der Schritt der Gruppenbildung folgendes umfasst: einen Schritt, um einen ersten Schätzwert für eine Position eines Übergangspunktes in einem Teilbereich zu bestimmen, und, ausgehend von dem genannten ersten Schätzwert, einen Schritt zur genaueren Bestimmung eines Übergangspunktes.

4. Bildverarbeitungsverfahren nach Anspruch 2 oder 3, **dadurch gekennzeichnet, dass** der Schritt der Zuordnung von Gewichtungen folgendes umfasst:
Berechnung (20) der Körnigkeit des Bildes,
Berechnung (19) der Höhe des Übergangs und
Berechnung (18) der genannten Gewichtungen, die von der Körnigkeit des Bildes und der Höhe des Übergangs für die entsprechenden Übergangspunkte abhängen.

5. Bildverarbeitungsverfahren nach einem der Ansprüche 1 bis 4, **dadurch gekennzeichnet, dass** der Schritt der Zuordnung von Gewichtungen folgendes umfasst:
Berechnung (15, 16) von einer ersten und einer zweiten räumlichen Ableitung von Helligkeitswerten nahe den entsprechenden Übergangspunkten und
Verwendung der wesentlichen Übereinstimmung eines Maximums der genannten ersten Ableitung mit einer Helligkeitsschwankung und mit einem Maximum der genannten zweiten Ableitung auf der peripheren Seite der genannten Helligkeitsschwankung zum Auswählen der Position der genannten Helligkeitsschwankung als Übergangspunkt, und Zuordnen der genannten Gewichtung auf der Grundlage der genannten Übereinstimmung.

6. Bildverarbeitungsverfahren nach einem der Ansprüche 1 bis 4, **dadurch gekennzeichnet, dass** der Schritt der Zuordnung von Gewichtungen folgendes umfasst:
Berechnung von ersten räumlichen Ableitungen von Helligkeitswerten, die peripher zu den entsprechenden Übergangspunkten liegen, Berechnung (13) eines entsprechenden mittleren Helligkeitswertes von Pixeln zur Mitte der genannten Übergangspunkte hin, Berechnung der entsprechenden Verhältnisse der genannten entsprechenden ersten räumlichen Ableitungen und der genannten entsprechenden mittleren Helligkeitswerte und Zuordnung der genannten Gewichtungen auf der Grundlage der genannten Verhältnisse.

7. Bildverarbeitungsverfahren nach einem der Ansprüche 1 bis 6, **dadurch gekennzeichnet, dass** dem Schritt der Zuordnung von Gewichtungen ein Schritt der Punktauswahl zum Auswählen eines Übergangspunktes folgt, der die höchste Gewichtung in einem Teilbereich aufweist, und nur die genannten ausgewählten Übergangspunkte in der genannten ausgewählten Gruppe beibehalten werden.

8. Bildverarbeitungsverfahren nach einem der Ansprüche 1 bis 7, **dadurch gekennzeichnet, dass** dem Schritt der Kurvenanpassung (21) ein Schritt der erneuten Zuordnung eines Wertes folgt, in dem einer Gewichtung eines Übergangspunktes erneut ein Wert in Abhängigkeit von einem Abstand zwischen dem genannten Übergangspunkt und einer durch die Gruppe, zu der der genannte Übergangspunkt gehört, gezogenen Kurve zugeordnet wird, und dass der Schritt der Kurvenanpassung wiederholt wird, wobei die genannte erneut zugeordnete Gewichtung eingesetzt wird.

9. Bildverarbeitungsverfahren nach einem der Ansprüche 1 bis 8, **dadurch gekennzeichnet, dass** dem Schritt der Randbestimmung (60) ein Schritt der Randüberprüfung (22) folgt, der die Berechnung eines Zuverlässigkeitsparameters für einen ermittelten Rand und den Vergleich des genannten Zuverlässigkeitsparameters mit einem vorher festgelegten Schwellenwert umfasst.

10. Bildverarbeitungsverfahren nach Anspruch 9, **dadurch gekennzeichnet, dass** der genannte Zuverlässigkeitsparameter für eine Vielzahl von Übergangspunkten berechnet wird, und dass in einer Situation, in der der Vergleich des genannten Zuverlässigkeitsparameters mit dem genannten vorher festgelegten Referenzwert die Existenz des genannten Teilbereichs, der einem Röntgenabsorptionsfilter entspricht, für weniger Übergangspunkte anzeigt als eine vorher festgelegte Referenzanzahl, der Schritt der Randüberprüfung durch den Vergleich der Pixelwerte von Pixelansammlungen auf verschiedenen Seiten der Kurve ergänzt wird.

11. Bildverarbeitungsverfahren nach Anspruch 10, **dadurch gekennzeichnet, dass** der genannte Zuverlässigkeitsparameter für eine Vielzahl von Übergangspunkten berechnet wird, und dass in einer Situation, in der der Vergleich des genannten Zuverlässigkeitsparameters mit dem genannten vorher festgelegten Referenzwert für weniger Übergangspunkte, als einer vorher festgelegten Referenzanzahl entsprechend, auf die Existenz des genannten Teilbereichs, der einem Röntgenabsorptionsfilter entspricht, hinweist, der Schritt der Randüberprüfung ergänzt wird durch die Bestimmung einer Größe der ersten räumlichen Ableitung von Helligkeitswerten von Pixeln in dem Bild zwischen der Kurve und dem Rand des Bildes, wobei die genannte Ableitung in der Richtung von der Kurve zum Rand des Bildes hin negativ ist, und durch den Vergleich der genannten Größe mit einem vorher festgelegten Referenzwert.

12. Bildverarbeitungsverfahren nach einem der Ansprüche 1 bis 11, **dadurch gekennzeichnet, dass** die Verarbeitung folgendes umfasst:
Auswählen des dem Röntgenabsorptionsfilter entsprechenden Teilbereichs als Teil des Bildes zwischen dem Rand und einer Peripherie des Bildes.

13. Bildverarbeitungsverfahren nach Anspruch 12, **dadurch gekennzeichnet, dass** die Verarbeitung folgendes umfasst:
Entfernen zumindest eines Teils des genannten Teilbereichs aus dem Bild.

14. Bildverarbeitungsverfahren nach Anspruch 12, **dadurch gekennzeichnet, dass** die Verarbeitung folgendes umfasst:
Zuordnung von weitgehend einheitlichen Werten zu den Pixeln in dem genannten Teilbereich des Bildes.

15. Bildprozessor zum Verarbeiten eines durch Röntgenbestrahlung gewonnenen Bildes, das einen Teilbereich enthält, das einem Röntgenabsorptionsfilter entspricht, **dadurch gekennzeichnet, dass** der Prozessor folgendes umfasst:
ein Bauteil zum Bestimmen eines Randes des Teilbereichs gemäß einem Verfahren nach einem der Ansprüche 1 bis 11 und
ein Bauteil zum Verändern des Bildes durch selektives Verarbeiten des Teilbereichs.

16. Röntgenuntersuchungsvorrichtung, die einen zwischen einem Röntgendetektor und einer Röntgenstrahlenquelle zum Bestrahlen eines Gegenstandes angeordneten Röntgenabsorptionsfilter umfasst, um ein Röntgenbild auf dem Röntgendetektor zu bilden, **dadurch gekennzeichnet, dass** die Vorrichtung einen Bildprozessor nach Anspruch 15 und einen mit dem Röntgendetektor gekoppelten Eingangsport umfasst.

## Revendications

1. Procédé de traitement d'images pour traiter une image formée par radiographie, l'image contenant une portion correspondant à un filtre d'absorption de rayons X, ledit procédé de traitement d'images comprenant :
la détermination d'un bord de ladite portion ;
la modification de l'image en traitant de manière sélective ladite portion ;
**caractérisé en ce que** la détection du bord comprend :
une étape de regroupement pour sélectionner un groupe de points de transition (3) dans l'image comportant une luminosité supérieure vers le centre de l'image et une luminosité inférieure vers la périphérie de l'image, des pondérations étant attribuées auxdits points de transition et une pondération pour un point de transition dépendant de manière décroissante d'une distance entre ledit point de transition et la périphérie de l'image, et
une étape d'ajustement de courbe (21) pour ajuster une courbe (60) à travers ledit groupe de points de transition, ladite courbe formant une représentation dudit bord.

2. Procédé de traitement d'images suivant la revendication 1, **caractérisé en ce que** l'étape de regroupement comprend :
une étape de sélection de région (11) pour sélectionner une région d'intérêt (1) s'étendant à partir d'une périphérie de l'image ;
une étape de division (12) pour diviser ladite région d'intérêt (1) en sections (2), et
une étape d'attribution de pondération (18) pour attribuer les pondérations auxdits points de transition (3), une pondération pour un point de transition dépendant de particularités d'image autour dudit point de transition.

3. Procédé de traitement d'images suivant la revendication 1 ou 2, **caractérisé en ce que** l'étape de regroupement comprend une étape de première estimation pour déterminer une première estimation pour une position d'un point de transition dans une section et une étape de précision pour déterminer un point de transition à partir de ladite première estimation.

4. Procédé de traitement d'images suivant la revendication 2 ou 3, **caractérisé en ce que** l'étape d'attribution de pondération comprend :
le calcul (20) d'une irrégularité d'image ;
le calcul (19) d'une hauteur de transition, et
le calcul (18) desdites pondérations en fonction de l'irrégularité d'image et de la hauteur de transition pour des points de transition respectifs.

5. Procédé de traitement d'images suivant l'une quelconque des revendications 1 à 4, **caractérisé en ce que** l'étape d'attribution de pondération comprend :
le calcul (15,16) d'une première et d'une deuxième dérivées spatiales de valeurs de luminosité à proximité de points de transition respectifs, et
l'utilisation du caractère concourant significatif d'un maximum de ladite première dérivée avec une variation de luminosité et avec un maximum de ladite deuxième dérivée sur le côté périphérique de ladite variation de luminosité pour sélectionner la position de ladite variation de luminosité sous la forme d'un point de transition et l'attribution de ladite pondération sur la base de ladite simultanéité.

6. Procédé de traitement d'images suivant l'une quelconque des revendications 1 à 4, **caractérisé en ce que** l'étape d'attribution de pondération comprend :
le calcul de premières dérivées spatiales de valeurs de luminosité périphériques à des points de transition respectifs, le calcul (13) de valeurs de luminosité moyennes respectives de pixels vers le centre par rapport auxdits points de transition, le calcul de rapports respectifs desdites premières dérivées spatiales respectives et desdites valeurs de luminosité moyennes respectives et l'attribution desdites pondérations sur la base desdits rapports.

7. Procédé de traitement d'images suivant l'une quelconque des revendications 1 à 6, **caractérisé en ce que** l'étape d'attribution de pondération est suivie d'une étape de sélection de point pour sélectionner un point de transition comportant la pondération la plus élevée dans une section et ne conserver que lesdits points de transition sélectionnés dans ledit groupe sélectionné.

8. Procédé de traitement d'images suivant l'une quelconque des revendications 1 à 7, **caractérisé en ce que** l'étape d'ajustement de courbe (21) est suivie d'une étape de réattribution pour réattribuer une valeur à une pondération d'un point de transition en fonction d'une distance entre ledit point de transition et une courbe ajustée à travers le groupe auquel ledit point de transition appartient, et **en ce que** l'étape d'ajustement de courbe est répétée en utilisant ladite pondération réattribuée.

9. Procédé de traitement d'images suivant l'une quelconque des revendications 1 à 8, **caractérisé en ce que** la détermination du bord (60) est suivie d'une étape de vérification du bord (22) comprenant le calcul d'un paramètre de fiabilité pour un bord déterminé et la comparaison dudit paramètre de fiabilité avec un niveau de seuil prédéterminé.

10. Procédé de traitement d'images suivant la revendication 9, **caractérisé en ce que**, pour une pluralité de points de transition, ledit paramètre de fiabilité est calculé et, dans une situation où la comparaison dudit paramètre de fiabilité audit niveau de référence prédéterminé indique la présence de ladite portion correspondant à un filtre d'absorption de rayons X pour moins de points de transition qu'un nombre de référence prédéterminé, l'étape de vérification du bord est complétée par la comparaison de valeurs de pixel d'ensembles de pixels sur différents côtés de la courbe.

11. Procédé de traitement d'images suivant la revendication 10, **caractérisé en ce que**, pour une pluralité de points de transition, ledit paramètre de fiabilité est calculé et, dans une situation où la comparaison dudit paramètre de fiabilité audit niveau de référence prédéterminé indiquait la présence de ladite portion correspondant à un filtre d'absorption de rayons X pour moins de points de transition qu'un nombre de référence prédéterminé, l'étape de vérification de bord est complétée par la détermination d'une grandeur d'une première dérivée spatiale de valeurs de luminosité de pixels dans l'image entre la courbe et la périphérie de l'image, ladite dérivée étant négative dans la direction allant de la courbe à la périphérie de l'image, et par la comparaison de ladite grandeur à une valeur de référence prédéterminée.

12. Procédé de traitement d'images suivant l'une quelconque des revendications 1 à 11, **caractérisé en ce que** le traitement comprend :
la sélection de la portion correspondant au filtre d'absorption de rayons X comme une partie de l'image entre le bord et une périphérie de l'image.

13. Procédé de traitement d'images suivant la revendication 12, **caractérisé en ce que** le traitement comprend :
l'élimination d'au moins une partie de ladite portion de l'image.

14. Procédé de traitement d'images suivant la revendication 12, **caractérisé en ce que** le traitement comprend :
l'attribution de valeurs pratiquement uniformes à des pixels dans ladite portion de l'image.

15. Dispositif de traitement d'images pour traiter une image formée par radiographie, laquelle contient une portion correspondant à un filtre d'absorption de rayons X, **caractérisé en ce que** le dispositif de traitement comprend :
un dispositif pour déterminer un bord de la portion selon un procédé suivant l'une quelconque des revendications 1 à 11, et
un dispositif pour modifier l'image en traitant de manière sélective la portion.

16. Appareil d'examen aux rayons X comprenant un filtre d'absorption de rayons X entre un détecteur de rayons X et une source de rayons X pour irradier un objet pour former une radiographie sur le détecteur de rayons X, **caractérisé en ce que** l'appareil comprend un dispositif de traitement d'images suivant la revendication 15 et comportant une entrée couplée au détecteur de rayons X.
